Europäisches Patentamt

European Patent Office

Office européen des brevets

(19)

(11) **EP 0 580 527 B2**

(12) **NOUVEAU FASCICULE DE BREVET EUROPEEN**

(45) Date de publication et mention de la décision concernant l'opposition:
**27.06.2001 Bulletin 2001/26**

(45) Mention de la délivrance du brevet:
**29.10.1997 Bulletin 1997/44**

(21) Numéro de dépôt: **93420298.7**

(22) Date de dépôt: **07.07.1993**

(51) Int Cl.⁷: $C07C\ 219/08$, $C07C\ 213/06$, $C07C\ 231/14$, $D06M\ 13/463$, $B01F\ 17/18$

(54) **Compositions tensioactives cationiques, à base de mono ou polyakyl ester et/ou amido ammonium, et leurs procédés de préparation**

Kationische tensioaktive Zusammensetzungen auf der Basis von Mono- oder Polyalkylester und/oder Amide von Ammoniumverbindungen und Verfahren zu ihrer Herstellung

Cationic surfactant compositions derived from mono or polyalkyl esters and/or amides of ammonium compounds and processes for their preparation

(84) Etats contractants désignés:
**AT BE CH DE DK ES FR GB GR IE IT LI LU MC NL PT SE**

(30) Priorité: **17.07.1992 FR 9209067**

(43) Date de publication de la demande:
**26.01.1994 Bulletin 1994/04**

(73) Titulaire: **STEPAN EUROPE 38340 Voreppe (FR)**

(72) Inventeurs:
• **Godefroy, Lionel**
  **F-38430 Moirans (FR)**
• **Storet, Yvan**
  **F-38113 Veurey Voroize (FR)**

(74) Mandataire: **Guerre, Dominique et al Cabinet Germain et Maureau, 12, rue Boileau, BP 6153 69466 Lyon Cedex 06 (FR)**

(56) Documents cités:
| | |
|---|---|
| EP-A- 0 075 168 | EP-A- 0 284 036 |
| WO-A-91/17974 | DE-A- 1 619 058 |
| DE-A- 2 430 140 | DE-A- 2 641 286 |
| US-A- 2 589 674 | US-A- 2 935 474 |
| US-A- 3 915 867 | |

Remarques:
Le dossier contient des informations techniques présentées postérieurement au dépôt de la demande et ne figurant pas dans le présent fascicule.

**Description**

La présente invention concerne des compositions tensioactives cationiques comprenant notamment des agents tensioactifs, analogues à ceux décrits dans la demande de brevet français 91 16479 du 31.12.1991 au nom de la présente Demanderesse.

Conformément à la demande de brevet français 91 16479 du 31.12.91, on a déjà décrit et proposé différents agents tensioactifs cationiques, consistant en des sels d'ammonium quaternaire, obtenus de manière générale à partir de la condensation entre, d'une part des acides gras saturés ou insaturés, ou des dérivés desdits acides, choisis dans le groupe comprenant les esters, anhydrides et chlorures d'acide, et d'autre part au moins une amine tertiaire. Plus précisément, les produits de condensation retenus selon cette invention présentent, avant quaternisation, les caractéristiques suivantes :

- les chaînes alkyles de l'amine tertiaire comportent ensemble de 4 à 12 atomes de carbone
- les chaînes alkyles de l'amine tertiaire comportent ensemble de 1 à 4 fonctions choisies chacune dans le groupe comprenant les fonctions hydroxyle et amine
- le rapport molaire des acides gras par rapport à l'amine tertiaire est compris entre 1,85 et 1,40
- les acides gras présentent chacun une chaîne hydrocarbonée dont le nombre d'atomes de carbone est compris entre 5 et 23.

Après quaternisation du produit de condensation obtenu comme précédemment, on obtient un composé cationique répondant à la formule moléculaire (I) suivante

$$\left[ R_4 - N \begin{array}{l} C_nH_{(2n+1-p-x)}(Y_1COR_1)_p(Y_{1'}H)_x \\ C_{n'}H_{(2n'+1-q-y)}(Y_2COR_2)_q(Y_{2'}H)_y \\ C_{n''}H_{(2n''+1-r-z)}(Y_3COR_3)_r(Y_{3'}H)_z \end{array} \right]^+ \quad X^-$$

dans laquelle:

- n, n', n" sont des nombres entiers non nuls
- p, q, r, x, y, z sont des nombres entiers ou zéro
- rapportée à une mole, la moyenne de la somme de n, n' et n" est comprise statistiquement entre 4 et 12
- rapportée à une mole, la moyenne statistique de la somme de p, q, r, x, y et z est comprise statistiquement entre 1 et 4
- rapportée à une mole, la moyenne de la somme de p, q et r est comprise entre 1,85 et 1,40
- $Y_1$, $Y_{1'}$, $Y_2$, $Y_{2'}$, $Y_3$, $Y_{3'}$ sont des groupes fonctionnels choisis chacun parmi les groupes oxy, et NH
- $R_1$, $R_2$, $R_3$ sont respectivement une longue chaîne hydrocarbonée alkyle ou alkényle, ayant 5 à 23 atomes de carbone
- $R_4$ est une courte chaîne alkyle ayant de 1 à 5 atomes de carbone
- X est un contre-ion choisi notamment parmi les halogénures, les carbonates, et les phosphates et les sulfates

De tels agents tensioactifs peuvent avoir différentes applications, en mélange ou non avec un ou plusieurs solvants, en mélange ou non avec d'autres additifs, en tant que détergents, agents assouplissants, notamment assouplissants textiles dilués ou concentrés, conditionneurs, agents antistatiques, lubrifiants, après-shampooings, etc... Ces applications et leurs modalités pratiques sont notamment décrites dans la demande de brevet français précitée, à laquelle on se référera utilement.

De manière générale, le produit de condensation, précédant la réaction de quaternisation, est obtenu en faisant réagir des acides gras répondant respectivement aux formules $R_1COOH$, $R_2COOH$, $R_3COOH$, ou leurs dérivés esters, anhydrides et chlorures, avec au moins une amine tertiaire répondant à la formule (II) :

$$N \begin{cases} C_nH_{(2n+1-p-x)}(Y_1H)_p(Y_1\cdot H)_x \\ C_{n'}H_{(2n'+1-q-y)}(Y_2H)_q(Y_2\cdot H)_y \\ C_{n''}H_{(2n''+1-r-z)}(Y_3H)_r(Y_3\cdot H)_z \end{cases} \quad (II)$$

dont les lettres et les indices sont identiques à ceux de la formule (I), la concentration molaire globale en acides gras étant comprise entre 1,85 et 1,40 fois la concentration en amines tertiaires. Puis, une fois ce produit de condensation obtenu, on le quaternarise avec un agent alkylant choisi parmi les halogénures, les sulfates, les phosphates et carbonates d'alkyle, en présence ou non d'un solvant.

Par exemple, ces produits de condensation peuvent être préparés par réaction d'une fraction d'acides gras sur la triéthanolamine (TEA). Mais ces mêmes produits peuvent être préparés par réaction d'esters méthyliques d'acides gras sur une amine tertiaire, conformément au document US-C-3 915 867.

Selon EP-0 284 036, il est décrit un procédé pour préparer sous forme de sel d'ammonium quaternaire un produit du même type que ceux précités, obtenu :

- par condensation entre une fraction d'acides gras sous forme de triglycérides ou triesters de glycérol et une amine tertaire répondant à la formule (II) ci-dessus, dans laquelle :

  - n, n', n" sont des nombres entiers non nuls, dont la moyenne de la somme est au moins égale à 4,
  - p, q, r, x, y, z sont des nombres entiers ou zéro,
  - $Y_1$, $Y_{1'}$, $Y_2$, $Y_{2'}$, $Y_3$, $Y_{3'}$ sont des groupes fonctionnels oxy

    lesdits acides gras présentant une chaîne hydrocarbonée saturée ou insaturée dont le nombre d'atomes de carbone est compris entre 5 et 23, et le rapport molaire dudit triester par rapport à l'amine tertiaire étant au moins égal à 1/3,
- puis par quaternisation du produit de condensation ainsi obtenu avec un agent alkylant, en présence ou non d'un solvant.

Les conditions opératoires de la réaction de condensation prévoient l'utilisation possible d'un catalyseur, et une longue durée de réaction pouvant atteindre 20 heures.

Les sels d'ammonium quaternaire obtenus selon les procédés précités nécessitent, pour certaines applications, différents additifs dont la nature chimique peut être voisine des composés selon la formule (I), notamment pour faciliter leurs formulations avec d'autres produits ou solvants, ou pour renforcer leurs propriétés. L'incorporation ou adjonction de ces additifs rend plus complexe, plus difficile et plus long, le protocole de préparation ou formulation du produit final, c'est-à-dire prêt à l'emploi.

Conformément à la présente invention, et pour certaines applications, telles que assouplissants textiles dilués ou concentrés, après-shampooings ou conditionneurs pour cheveux, on a découvert que l'on pouvait obtenir directement, et donc plus économiquement, une composition tensioactive cationique prête à l'emploi, en choisissant une réaction de condensation particulière, à savoir une transestérification et/ou transester-amidification entre au moins un acide gras saturé ou insaturé sous forme de triester de glycérol et au moins une amine tertiaire, volontairement limitée dans son taux réactionnel, et par conséquent partielle.

Plus précisément, dans la réaction de transestérification et/ou transesteramidification, le rapport molaire des acides gras à l'amine tertiaire est compris entre 1 et 3 à l'exception des valeurs comprises entre 1,40 et 1,85.

Cette réaction de transestérification et/ou transester-amidification est suivie d'une réaction de quaternisation, entre le produit de condensation et un agent alkylant, avec un taux réactionnel volontairement limité, et donc partiel. En cas de quaternisation partielle de l'amine tertiaire résiduelle, cette dernière non quaternisée est avantageusement neutralisée ou salifiée avec des acides minéraux ou organiques. Le mélange finalement obtenu peut être additionné de tout solvant approprié et d'additifs secondaires.

Préférentiellement, selon l'invention, le milieu réactif de départ comprend en outre des acides gras et les réactions de transestérification et/ou transestéramidification s'accompagnent de réaction d'estérification et/ou d'amidification.

En conséquence, ledit produit de condensation peut aussi être obtenu par réaction d'au moins un acide gras sous forme libre avec ladite amine tertaire de façon concomitante à la réaction de la même amine avec le triester de glycérol et/ou il peut se former un autre produit de condensation entre au moins un acide gras sous forme libre et ladite amine tertaire.

Par "directement", selon l'invention, on entend un processus réactionnel permettant d'obtenir un produit ne nécessitant plus de transformation chimique ultérieure pour ses applications.

De cette manière, après quaternisation, on obtient une composition tensioactive, dont le produit de condensation sous forme de sel d'ammonium quaternaire représente au moins 60 % en poids de ladite composition, le solde de cette dernière comprenant au moins des produits secondaires de la réaction de transestérification et/ou transestéramidification, et/ou des réactions d'estérification et/ou amidification, dont monoesters, diesters et triesters de glycérol, glycérol, et des produits n'ayant pas réagi.

Au sens de l'invention, une transestérification est la réaction d'un triglycéride ou triester de glycérol avec les groupements fonctionnels oxy de l'amine tertiaire, une transestéramidification est la réaction d'un triglycéride avec les groupements fonctionnels $NH_2$ de l'amine tertaire, une estérification est la réaction des acides gras sous forme libre avec les groupements oxy de l'amine, une amidification est la réaction des acides gras sous forme libre avec les groupements $NH_2$ de l'amine tertiaire.

Une telle composition s'est révélée directement utilisable, au moins aussi efficace que lorsque le rendement de la réaction de condensation est maximal ou lorsque le produit de condensation est obtenu à partir d'acides gras seuls ou d'esters méthyliques d'acides gras, et donc économique à préparer, puisqu'en particulier on a découvert que les produits secondaires précités sont en grande partie actifs vis-à-vis des applications considérées, et apportent des effets complémentaires, tels qu'un effet lubrifiant, adoucissant, épaississant, hydratant, tout en jouant pour partie le rôle d'un solvant.

Le procédé selon l'invention se prête à une industrialisation dans des conditions économiques, d'une part parce qu'il utilise une matière première peu élaborée et abondante, en l'occurrence au moins un triester de glycérol, ou triglycéride tel que huile de palme, et d'autre part parce qu'au moins une étape réactionnelle est limitée, et donc plus courte.

Les compositions tensioactives selon l'invention présentent au surplus l'avantage d'être entièrement biodégradables.

La présente invention comporte les caractéristiques techniques complémentaires suivantes :

- le solde de la composition ou du mélange tensioactif comprend des produits secondaires de la réaction partielle, c'est-à-dire non complète de quaternisation du produit de condensation, à savoir des alkyl-esters (et/ou amido) amines non quaternisées mais susceptibles d'être salifiées par un acide minéral ou organique; certains de ces produits se sont révélés en effet actifs, et pour certains d'entre eux tensio-actifs, de telle sorte qu'il peut être intéressant de limiter ou contrôler la réaction de quaternisation
- l'agent alkylant est préférentiellement choisi parmi les halogénures d'alkyle ou d'alkylbenzyle, tels que le chlorure de méthyle ou le chlorure de benzyle, les sulfates d'alkyle, du type polyalkylestersulfates, tels que les diméthyl ou diéthylsulfates, les phosphates d'alkyle, et les carbonates d'alkyle, tels que le diméthylcarbonate
- les acides gras du triester de glycérol comprennent préférentiellement au moins 50 % en moles d'acides linéaires en $C_{16}$ à $C_{18}$
- les acides gras du triester de glycérol présentent préférentiellement une insaturation, exprimée en g d'iode pour 100 g d'acides gras, au plus égale à 120, et notamment comprise entre 30 et 60
- l'étape de transestérification et/ou transestéramidification, et éventuellement coestérification ou coestéramidification, s'effectue ou non en présence de catalyseurs usuels, notamment acides ou basiques
- la composition ou mélange tensioactif comprend ou non des antioxydants, utilisés lors de l'étape de transestérification et/ou transesteramidification, notamment du butylhydroxytoluène ou du butylhydroxyanisol
- la composition ou mélange tensioactif comprend ou non un agent décolorant, réducteur ou oxydant
- la composition ou mélange tensioactif comprend ou non différents agents modificateurs ou protecteurs, tels que acides citriques, acides tartriques, hydrures simples ou mixtes
- la composition ou mélange tensioactif comprend ou non un agent complexant
- un solvant complémentaire, notamment du type alcool, glycol ou ester, peut être ajouté à la composition, pour en modifier la présentation et certaines caractéristiques
- s'agissant de l'amine tertiaire de formule (II), utilisée dans le processus réactionnel selon l'invention, la somme des indices p, q, r, x, y et z de l'amine tertiaire est au plus égale à 4
- l'amine tertiaire de départ est préférentiellement polyfonctionnelle, c'est-à-dire comporte plusieurs fonctions hydroxyle ou amine primaire ou secondaire, et est choisie notamment parmi le N,N diéthylamino-2,3 propane diol, le N,N diméthylamino-2,3 propane diol, la N éthyldiéthanolamine, et la triéthanolamine
- l'amine tertiaire est aussi éventuellement monofonctionnelle et peut être aussi associée à une amine polyfonctionnelle, c'est-à-dire comporte une seule fonction hydroxyle ou amine primaire ou secondaire, et est notamment choisie dans le groupe comprenant :

  la NN diméthylaminopropylamine

la NN diéthylaminopropylamine
la NN diméthylaminoéthanolamine
la NN diéthylaminoéthanolamine
la NN diméthylaminoisopropanolamine
la NN diéthylaminoisopropanolamine

dans ce cas, avantageusement le rapport amine tertiaire polyfonctionnelle/amine tertiaire monofonctionnelle est compris entre 2 et 200

- les acides gras retenus sous forme de triester de glycérol sont notamment des huiles ou graisses d'origine naturelle, raffinées ou non, partiellement, totalement ou pas du tout hydrogénées, telles que le suif, le saindoux, l'huile de coprah, l'huile de palme, l'huile de soja, l'huile de poisson, l'huile de colza, et l'huile de tournesol.

**EXEMPLE 1 :**

Un mélange de 538 g d'huile de palme partiellement hydrogénée et de 150 g de triéthanolamine ou TEA est chauffé sous atmosphère d'azote pendant au moins 4 h à 140°C, en présence de 1,5 % de méthylate de sodium, jusqu'à une teneur en TEA libre inférieure à 6 % en poids.

Le milieu réactionnel est ensuite traité par 126 g de $Me_2SO_4$. Le mélange d'ammoniums quaternaires formés est dilué dans de l'alcool isopropylique pour donner un extrait sec de 80 à 85 %, puis décoloré à l'eau oxygénée ou un chlorite.

L'agent tensioactif contenu dans le mélange ainsi préparé répond statistiquement à la formule (I) dans laquelle :

- n+n'+n"=6
- p+q+r+x+y+z=3
- $Y_1$, $Y_{1'}$, $Y_2$, $Y_{2'}$, $Y_3$, $Y_{3'}$ sont des groupes oxy
- $R_4$ = $CH_3$
- $X^-$ = $CH_3 SO_4$

**EXEMPLE 2 :**

Un mélange de 294 g d'huile de palme, de 5,1 g de diméthylaminopropylamine, de 73 g de triéthanolamine et 0,2 g d'acide phosphoreux est chauffé sous balayage d'azote à 140°C pour distiller l'eau de réaction formée. La température est progressivement augmentée à 190°C puis maintenue pendant au moins 4h. A un taux de conversion minimum de 75 % de la triéthanolamine, le milieu réactionnel est refroidi à 60°C, puis quaternisé par 62 g de diméthylsulfate (DMS).

48 g d'isopropanol (IPA) sont ajoutés pour ajuster l'extrait sec à 90 %. La matière active finale, mesurée selon la norme AFNOR NFT 73-320 (ou ISO 2871-1 de mai 1989) est alors de 0,81 mE/g.

L'agent tensioactif contenu dans le mélange ainsi préparé répond statistiquement à la formule (I) dans laquelle :

- n+n'+n"=6 ou 5
- p + q + r + x + + y + z = 3 ou 1
- $Y_1$, $Y_{1'}$, $Y_2$, $Y_{2'}$, $Y_3$, $Y_{3'}$ sont des groupes oxy ou NH
- $R_4$ = $CH_3$
- $X^-$ = $CH_3SO_4$

**EXEMPLE 3 :**

Un mélange de 425 g d'huile de palme, de 110 g de diéthylamino-3 propanediol 1,2 est porté à 190°C sous agitation et atmosphère d'azote pendant 8 h.

A un taux de conversion minimum de 75 % de l'aminodiol, le milieu réactionnel est refroidi à 60°C puis quaternisé par 89 g de DMS. L'ensemble est chauffé à 80°C pendant 1 h.

La matière active finale, mesurée selon la norme NFT 73-320 est alors de 0,87 mE/g.

L'agent tensioactif contenu dans le mélange ainsi préparé répond à la formule (I) dans laquelle :

- n+n'+n"=7
- p+q+r+x+y+z=2

- $Y_1$, $Y_{1'}$, $Y_2$, $Y_{2'}$, $Y_3$, $Y_{3'}$ sont des groupes oxy
- $R_4 = CH_3$
- $X^- = CH_3SO_4$

Les exemples 4 et 5 illustrent une préparation particulière de compositions de l'invention, selon laquelle le milieu réactif comprend en outre des acides gras sous forme libre.

**EXEMPLE 4 :**

Selon cet exemple, la fraction acide gras comprend 80% de triesters et 20% d'acides gras sous forme libre.
Un mélange de 491 g d'huile de palme (1,72 Eq), de 122,8 g d'acides gras de palme (0,45 Eq), de 154,7 g de triéthanolamine (1,04 Eq) et de 0,4 g d'hydroborure de sodium est porté à 190°C sous agitation et atmosphère d'azote pendant 4 heures au cours desquelles 7 cm³ d'eau environ sont récupérés par distillation sous pression atmosphérique.
A un taux de conversion d'environ 75 % de la triéthanolamine, le milieu réactionnel est refroidi à 60°C puis quaternisé par 126 g de DMS (1,0 Eq). L'ensemble est chauffé à 80°C pendant 1 h.
99,5 g d'isopropanol sont ajoutés pour ajuster l'ester sec à 90%. La matière active finale, mesurée selon la norme AFNOR NFT 73-320 (ou ISO 2871-1 de mai 1989) est alors de 0,86 mEq/g.
L'agent tensioactif (hors sous-produit) contenu dans le mélange ainsi préparé répond à la formule (I) dans laquelle :

- n+n'+n"=6
- p+q+r+x+y+z=3
- $Y_1$, $Y_{1'}$, $Y_2$, $Y_{2'}$, $Y_3$, $Y_{3'}$ sont des groupes oxy
- $R_4 = CH_3$
- $X^- = CH_3SO_4^-$

**EXEMPLE 5 :**

Selon cet exemple, la fraction acide gras comprend 60% de triesters et 40% d'acides gras sous forme libre.
Un mélange de 369 g d'huile de palme (1,29 Eq), de 246 g d'acides gras de palme (0,91 Eq), de 156,8 g de triéthanolamine (1,05 Eq) et de 0,38 g d'hydroborure de sodium est porté à 190°C sous agitation et atmosphère d'azote pendant 4 heures au cours desquelles 15 cm³ d'eau environ sont récupérés par distillation sous pression atmosphérique.
A un taux de conversion d'envrion 75 % de la triéthanolamine, le milieu réactionnel est refroidi à 60°C puis quaternisé par 128,5 g de DMS (1,02 Eq). L'ensemble est chauffé à 80°C pendant 1 h.
100 g d'isopropanol sont ajoutés pour ajuster l'ester sec à 90%. La matière active finale, mesurée selon la norme AFNOR NFT 73-320 (ou ISO 2871-1 de mai 1989) est alors de 0,88 mEq/g.
L'agent tensioactif (hors sous-produit) contenu dans le mélange ainsi préparé répond à la formule (I) dans laquelle :

- n+n'+n"=6
- p+q+r+x+y+z=3
- $Y_1$, $Y_{1'}$, $Y_2$, $Y_{2'}$, $Y_3$, $Y_{3'}$ sont des groupes oxy
- $R_4 = CH_3$
- $X^- = CH_3SO_4^-$

Les mélanges et compositions tensioactifs obtenus comme précédemment sont utilisables notamment pour la préparation d'assouplissants textiles aqueux, aussi bien sous faible concentration (5 % d'extraits secs environ par exemple), que sous forte concentration (15, 20, 25 % ou plus d'extraits secs), avec des viscosités similaires à celles usuellement rencontrées. Ces assouplissants peuvent être préparés, aussi bien sans additifs qu'avec des additifs usuels tels que le chlorure de calcium, un sel minéral ou organique approprié, un colorant, un parfum ou la majorité des additifs généralement rencontrés dans ce domaine d'application.
Pour évaluer les propriétés assouplissantes des compositions, une étude comparative a été menée avec 20 consommateurs entre 3 compositions comprenant respectivement les agents tensioactifs suivants :

- un produit de condensation quaternisé obtenu par réaction d'une fraction acide gras sous forme d'huile de palme et de triéthanolamine, similaire au produit de l'Exemple 1, mais le rapport acide gras/amine tertiaire est égal à 2,25

- un produit de condensation quaternisé obtenu par réaction entre une fraction acide gras de suif partiellement hydrogéné (Indice d'iode d'environ 40 g I$_2$/100g) et la triéthanolamine, le rapport acide gras/amine tertiaire étant égal à 1,86
- un produit de condensation quaternisé obtenu par réaction entre une fraction acide gras de palme et la triéthanolamine, le rapport acide gras/amine tertiaire étant égal à 1,90.

Tous ces produits ont été testés dilués, à la dose de 1,8 g de produit sec (10 h/105°C) par Kg de textiles sur des serviettes de coton neuves d'abord prélavées, puis lavées et enfin rincées, dans la même machine à laver ménagère traditionnelle.

Le produit obtenu à partir de triglycéride a été jugé identique à celui obtenu à partir d'acides gras de palme et bien supérieur à celui obtenu à partir d'acides gras de suif. Cette étude permet de montrer l'excellent rapport prix/ performance des produits obtenus à partir d'huile, ces produits étant les moins chers des trois, les plus faciles et les plus rapides à préparer et les seuls ne produisant quasiment pas de déchets lors des réactions de préparation.

**Revendications**

1. Composition tensioactive cationique comprenant, sous forme de sel d'ammonium quaternaire, au moins un produit de condensation entre, d'une part au moins un acide gras saturé ou insaturé, sous forme de triester de glycérol, et d'autre part au moins une amine tertiaire, les chaînes alkyle de l'amine tertiaire comportant ensemble de 4 à 12 atomes de carbone, et au moins un groupe fonctionnel, et l'acide gras présentant une chaîne hydrocarbonée dont le nombre d'atomes de carbone est compris entre 5 et 23, le rapport molaire dudit triester par rapport à l'amine tertiaire étant au moins égal à 1/3, caractérisée en ce que, les fonctions de l'amine tertiaire sont en nombre compris entre 1 et 4 et sont choisies chacune dans le groupe comprenant les fonctions hydroxyle et amine, et ledit produit de condensation est susceptible d'être obtenu directement par une transestérification et /ou transestéramidification volontairement limitée dans son taux réactionnel, puis quaternisation, le rapport molaire des acides gras par rapport à l'amine tertiaire étant compris entre 1 et 3, à l'exception des valeurs comprises entre 1,40 et 1,85, et en ce que ledit produit de condensation sous forme de sel d'ammonium quaternaire représente au moins 60 % en poids de ladite composition cationique, le solde de ladite composition comprenant au moins des produits secondaires susceptibles d'être obtenus par la réaction de transestérification et/ou transesteramidification, dont monoesters, diesters de glycérol, glycérol, et des produits n'ayant pas réagi.

2. Composition selon la revendication 1, caractérisée en ce que le produit de condensation est en outre obtenu par réaction d'au moins un acide gras sous forme libre avec ladite amine, de façon concomitante à la réaction de la même amine avec le triester de glycérol.

3. Composition selon la revendication 1 ou 2, caractérisée en ce qu'elle comprend un autre produit de condensation entre au moins un acide gras sous forme libre et ladite amine.

4. Composition selon la revendication 1, caractérisée en ce que le solde de ladite composition comporte aussi des produits secondaires susceptibles d'être obtenus par la réaction de quaternisation du produit de condensation, dont ledit produit de condensation non quaternisé.

5. Composition selon la revendication 1, caractérisée en ce que le solde de la composition comporte ladite amine tertiaire sous forme totalement ou partiellement salifiée.

6. Composition selon la revendication 1, caractérisée en ce que les acides gras sous la forme de triester de glycérol comprennent au moins 50 % en moles d'acides linéaires en C$_{16}$ à C$_{18}$.

7. Composition selon la revendication 1, caractérisée en ce que les acides gras sous la forme de triester de glycérol présentent une insaturation, exprimée en g d'iode pour 100 g d'acides gras, au plus égale à 120, et notamment comprise entre 30 et 60.

8. Procédé pour préparer directement un mélange tensioactif, selon lequel on fait réagir au moins des acides gras sous forme de triester de glycérol avec au moins une amine tertiaire répondant à la formule (II) :

$$N \begin{array}{l} C_nH_{(2n+1-p-x)}(Y_1H)_p(Y_1{}'H)_x \\ C_{n'}H_{(2n'+1-q-y)}(Y_2H)_q(Y_2{}'H)_y \\ C_{n''}H_{(2n''+1-r-z)}(Y_3H)_r(Y_3{}'H)_z \end{array}$$

dans laquelle :

- n, n', n" sont des nombres entiers non nuls,
- p, q, r, x, y, z sont des nombres entiers ou zéro,
- rapportée à une mole, la moyenne de la somme de n, n' et n" est comprise statistiquement entre 4 et 12,
- $Y_1$, $Y_1{}'$, $Y_2$, $Y_2{}'$, $Y_3$, $Y_3{}'$ sont des groupes fonctionnels, lesdits acides gras présentant une chaîne hydrocarbonée saturée ou insaturée dont le nombre d'atomes de carbone est compris entre 5 et 23,

puis on fait réagir le produit de condensation ainsi obtenu avec un agent alkylant choisi parmi les halogénures, les sulfates, les phosphates et carbonates d'alkyle, en présence ou non d'un solvant

caractérisé en ce que : dans la formule II :

- d'une part, rapportée à une mole, la moyenne de la somme de p, q, r, x, y et z est comprise statistiquement entre 1 et 4,
- et d'autre part, $Y_1$, $Y_1{}'$, $Y_2$, $Y_2{}'$, $Y_3$, $Y_3{}'$ sont des groupes fonctionnels choisis chacun parmi les groupes oxy et NH,

et en ce qu'on fait réagir partiellement l'amine tertiaire avec le triester de glycérol, selon une transestérification et/ou transesteramidification dont on limite volontairement le taux réactionnel, le rapport molaire des acides gras par rapport à l'amine tertiaire étant compris entre 1 et 3, à l'exception des valeurs comprises entre 1,40 et 1,85.

9. Procédé selon la revendication 8, caractérisé en ce qu'on fait en outre réagir lesdits acides gras sous forme libre avec ladite amine tertiaire.

10. Procédé selon la revendication 9, caractérisé en ce qu'on fait aussi réagir partiellement le produit de condensation avec l'agent alkylant.

11. Procédé selon la revendication 9, caractérisé en ce que l'amine tertaire résiduelle est partiellement quaternisée et que l'amine tertiaire non quaternisée est neutralisée avec des acides minéraux ou organiques.

12. Procédé selon la revendication 9, caractérisé en ce que l'amine tertiaire est monofonctionnelle, c'est-à-dire comporte une seule fonction hydroxyle ou amine primaire ou secondaire, et est notamment choisie dans le groupe comprenant

la NN diméthylaminopropylamine
la NN diéthylaminopropylamine
la NN diméthylaminoéthanolamine
la NN diéthylaminoéthanolamine
la NN diméthylaminoisopropanolamine
la NN diéthylaminoisopropanolamine

13. Procédé selon la revendication 12, caractérisé en ce qu'au moins une amine tertiaire polyfonctionnelle, c'est-à-dire comportant plusieurs fonctions hydroxyle ou amine primaire ou secondaire, est associée à l'amine tertiaire monofonctionnnelle, dans un rapport molaire amine tertiaire polyfonctionnelle/amine tertiaire monofonctionnelle compris entre 2 et 200.

14. Procédé selon la revendication 13, caractérisé en ce que l'amine tertiaire polyfonctionnelle est choisie parmi le N, N diéthylamino-2,3 propane diol, le N,N diméthylamino-2,3 propane diol, la méthyldiéthanolamine, l'éthyldiéthanolamine, et la triéthanolamine.

**Patentansprüche**

1. Kationische, tensioaktive Zusammensetzung, die, in Form eines quartären Ammoniumsalzes, zumindest ein Kondensationsprodukt aus einerseits zumindest einer gesättigten oder ungesättigten Fettsäure in Form eines Glycerintriesters und aus andererseits zumindest einem tertiären Amin enthält, wobei die Alkylketten des tertiären Amins zusammen 4 bis 12 Kohlenstoffatome und zumindest eine funktionelle Gruppe aufweisen, und wobei die Fettsäure eine Kohlenwasserstoffkette mit 5 bis 23 Kohlenstoffatomen aufweist, wobei das molare Verhältnis zwischen dem Triester und dem tertiären Amin zumindest gleich 1/3 ist, dadurch gekennzeichnet, daß die funktionellen Gruppen des tertiären Amins in einer Anzahl von 1 bis 4 vorhanden sind und jeweils ausgewählt sind aus der Gruppe enthaltend die Hydroxyl-oder die Aminfunktion, und wobei das Kondensationsprodukt direkt durch eine in ihrem Umsetzgrad willentlich begrenzte Transesterifizierung und/oder Transesteramidierung, gefolgt von einer Quarternisierung, erhalten wird, wobei das molare Verhältnis der Fettsäuren zum tertiären Amin im Bereich von 1 bis 3 liegt, mit Ausnahme der Werte von 1,40 bis 1,85, und daß das Kondensationsprodukt in Form eines quartären Ammoniumsalzes zumindest 60 Gew.-% der kationischen Zusammensetzung ausmacht, wobei der Rest der Zusammensetzung zumindest Sekundärprodukte aus der Transesterifizierungsreaktion und/oder der Transesteramidierungsreaktion aufweist, nämlichGlycerinmonoester, Glycerindiester, Glycerin und Produkte, die nicht reagiert haben.

2. Zusammensetzung nach Anspruch 1, dadurch gekennzeichnet, daß das Kondensationsprodukt außerdem durch Reaktion von zumindest einer Fettsäure in freier Form mit dem genannten Amin erhalten wird, und zwar begleitend zu der Reaktion desselben Amins mit dem Glycerintriester.

3. Zusammensetzung nach Anspruch 1 oder 2, dadurch gekennzeichnet, daß diese ein weiteres Kondensationsprodukt aus zumindest einer Fettsäure in freier Form und dem genannten Amin enthält.

4. Zusammensetzung nach Anspruch 1, dadurch gekennzeichnet, daß der Rest der Zusammensetzung auch Sekundärprodukte enthält, die aus der Quarternisierungsreaktion des Kondensationsproduktes erhalten werden können, nämlich von dem nicht quarternisierten Kondensationsprodukt.

5. Zusammensetzung nach Anspruch 1, dadurch gekennzeichnet, daß der Rest der Zusammensetzung das tertiäre Amin teilweise oder vollständig in Form eines Salzes enthält.

6. Zusammensetzung nach Anspruch 1, dadurch gekennzeichnet, daß die Fettsäuren in Form von Glycerintriester zumindest 50 Mol-% lineare $C_{16}$ - $C_{18}$-Säuren enthalten.

7. Zusammensetzung nach Anspruch 1, dadurch gekennzeichnet, daß die Fettsäuren in Form von Glycerintriester eine Unsättigung, ausgedrückt in g Jod pro 100 g Fettsäure, von höchstens gleich 120, und insbesondere zwischen 30 und 60 aufweisen.

8. Verfahren zum direkten Herstellen einer tensioaktiven Mischung, bei dem man zumindest Fettsäuren in Form von Glycerintriester mit zumindest einem tertiären Amin reagieren läßt, das der folgenden Formel (II) entspricht:

$$N \begin{cases} C_n H_{(2n+1-p-x)} & (Y_1 H)_p & (Y_{1'} H)_x \\ C_{n'} H_{(2n'+1-q-y)} & (Y_2 H)_q & (Y_{2'} H)_y \\ C_{n''} H_{(2n''+1-r-z)} & (Y_3 H)_r & (Y_{3'} H)_z \end{cases}$$

in der

- n, n', n'' ganze Zahlen, jedoch nicht Null bedeuten
- p, q, r, x, y, z ganze Zahlen oder Null bedeuten
- bezüglich eines Mols, der mittlere Summenwert von n, n' und n'' statistisch 4 bis 12 beträgt,
- $Y_1$, $Y_{1'}$, $Y_2$, $Y_{2'}$, $Y_3$, $Y_{3'}$ funktionelle Gruppen sind,

wobei die Fettsäuren eine gesättigte oder ungesättigte Kohlenwasserstoffkette aufweisen, deren Anzahl an Kohlenstoffatomen im Bereich von 5 bis 23 liegt,

man anschließend das so erhaltene Kondensationsprodukt mit einem Alkylierungsagens, ausgewählt aus den Alkylhalogeniden, den Alkylsulfaten, den Alkylphosphaten und den Alkylcarbonaten, in Gegenwart oder ohne ein Lösungsmittel reagieren läßt,

dadurch gekennzeichnet, daß     in der Formel II

- einerseits, bezüglich eines Mols, der mittlere Summenwert von p, q, r, x, y und z statistisch im Bereich von 1 bis 4 liegt, und
- andererseits $Y_1$, $Y_{1'}$, $Y_2$, $Y_{2'}$, $Y_3$, $Y_{3'}$ funktionelle Gruppen ausgewählt aus den Gruppen Oxy und NH sind,

und daß man das tertiäre Amin partiell mit dem Glycerintriester im Sinne einer Transesterifizierung und/oder einer Transesteramidierung reagieren läßt, bei der man den Umsetzgrad willentlich begrenzt, wobei das molare Verhältnis der Fettsäuren zum tertiären Amin im Bereich von 1 bis 3 liegt, mit Ausnahme der Werte von 1,40 bis 1,85.

9. Verfahren nach Anspruch 8, dadurch gekennzeichnet, daß man darüber hinaus die genannten Fettsäuren in freier Form mit dem genannten tertiären Amin reagieren läßt.

10. Verfahren nach Anspruch 9, dadurch gekennzeichnet, daß man auch partiell das Kondensationsprodukt mit dem Alkylierungsagens reagieren läßt.

11. Verfahren nach Anspruch 9, dadurch gekennzeichnet, daß das rückständige tertiäre Amin teilweise quarternisiert wird, und daß das nicht quarternisierte tertiäre Amin mit mineralischen oder organischen Säuren neutralisiert wird.

12. Verfahren nach Anspruch 9, dadurch gekennzeichnet, daß das tertiäre Amin monofunktionell ist, d.h. eine einzige Hydroxyl- oder eine einzige primäre oder sekundäre Aminfunktion trägt, und insbesondere ausgewählt ist aus der Gruppe bestehend aus

N,N-Dimethylaminopropylamin
N,N-Diethylaminopropylamin
N,N-Dimethylaminoethanolamin
N,N-Diethylaminoethanolamin
N,N-Dimethylaminoisopropanolamin
N,N-Diethylaminoisopropanolamin.

13. Verfahren nach Anspruch 12, dadurch gekennzeichnet, daß zumindest ein polyfunktionelles tertiäres Amin, d.h. eines, das zahlreiche primäre oder sekundäre Hydroxyl- oder Aminfunktionen enthält, mit einem monofunktionellen tertiären Amin assoziiert wird, und zwar in einem molaren Verhältnis von polyfunktionellem tertiärem Amin/monofunktionellem tertiärem Amin im Bereich von 2 bis 200.

14. Verfahren nach Anspruch 13, dadurch gekennzeichnet, daß das polyfunktionelle tertiäre Amin ausgewählt ist aus N,N-Diethylamino-2,3-propanol, N,N-Dimethylamino-2,3-propandiol, Methyldiethanolamin, Ethyldiethanolamin und Triethanolamin.

**Claims**

1. Cationic surface-active composition comprising, in the quaternary ammonium salt form, at least one condensation product between, on the one hand, at least one saturated or unsaturated fatty acid, in the glyceryl triester form, and, on the other hand, at least one tertiary amine, the alkyl chains of the tertiary amine together containing from 4 to 12 carbon atoms, and at least one functional group, and the fatty acid having a hydrocarbon chain in which the number of carbon atoms is between 5 and 23, the molar ratio of the said triester with respect to the tertiary amine being at least equal to 1/3, characterized in that, the functional groups of the tertiary amine are, in number, between 1 and 4 and are each chosen from the group comprising hydroxyl and amine functional groups, and the said condensation product is capable of being directly obtained by a partial transesterification and/or transesteramidification with a deliberately limited degree of reaction, and quaternization, the molar ratio of the fatty acids with respect to the tertiary amine being between 1 and 3, with the exception of the values between 1.40 and 1.85, and in that the said condensation product, in the quaternary ammonium salt form, represents at least 60 % by weight of the said cationic composition, the balance of the said composition comprising at least by-products capable of being obtained by the transesterification and/or transesteramidification reaction, including glyceryl monoesters or diesters, glycerol, and unreactive products.

2. Composition according to claim 1, characterized in that the condensation product is additionally obtained by reaction of at least one fatty acid, in the free form, with the said amine, concomitantly with the reaction of the same amine with the glyceryl triester.

3. Composition according to claim 1 or 2, characterized in that it comprises another product of condensation between at least one fatty acid, in the free form, and the said amine.

4. Composition according to claim 1, characterized in that the balance of the said composition also contains by-products capable of being obtained by the quaternization reaction of the condensation product, including the said non-quaternized condensation product.

5. Composition according to claim 1, characterized in that the balance of the composition comprises the said tertiary amine in the totally or partially salified form.

6. Composition according to claim 1, characterized in that the fatty acids in the glyceryl triester form comprise at least 50 mol % of linear $C_{16}$ to $C_{18}$ acids.

7. Composition according to claim 1, characterized in that the fatty acids in the glyceryl triester form have an unsaturation, expressed in g of iodine per 100 g of fatty acids, at most equal to 120 and especially between 30 and 60.

8. Process for directly preparing a surface-active mixture, according to which at least fatty acids in the glyceryl triester form are reacted with at least a tertiary amine corresponding to the formula (II):

$$N \begin{cases} C_nH_{(2n+1-p-x)}(Y_1H)_p(Y_1'H)_x \\ C_{n'}H_{(2n'+1-q-y)}(Y_2H)_q(Y_2'H)_y \\ C_{n''}H_{(2n''+1-r-z)}(Y_3H)_r(Y_3'H)_z \end{cases}$$

in which:

- n, n', n'' are non-zero integers
- p, q, r, x, y, z are integers or zero
- with respect to one mole, the mean of the sum of n, n' and n'' is statistically between 4 and 12
- $Y_1$, $Y_1'$, $Y_2$, $Y_2'$, $Y_3$, $Y_3'$ are functional groups,

the said fatty acids having a saturated or unsaturated hydrocarbon chain in which the number of carbon atoms is between 5 and 23,
and then the condensation product thus obtained is reacted with an alkylating agent chosen from alkyl halides, sulfates, phosphates and carbonates, in the presence or in the absence of a solvent
characterized in that:
in the formula II:

- on the one hand, relative to one mole, the mean of the sum of p, q, r, x, y and z is statistically between 1 and 4,
- and, on the other hand, $Y_1$, $Y_1'$, $Y_2$, $Y_2'$, $Y_3$, $Y_3'$ are functional groups each chosen from oxy and NH groups,

and in that the tertiary amine is partially reacted with the glyceryl triester, according to a transesterification and/or transesteramidification which degree of reaction is deliberately limited, the molar ratio of the fatty acids with respect to the tertiary amine being between 1 and 3, with the exception of the values between 1.40 and 1.85.

9. Process according to claim 8, characterized in that the said fatty acids, in the free form, are additionally reacted with the said tertiary amine.

**10.** Process according to claim 9, characterized in that the condensation product is also partially reacted with the alkylating agent.

**11.** Process according to claim 9, characterized in that the residual tertiary amine is partially quaternized and in that the non-quaternized tertiary amine is neutralized with inorganic or organic acids.

**12.** Process according to claim 9, characterized in that the tertiary amine is monofunctional, that is to say contains a single primary or secondary hydroxyl or amine functional group and is especially chosen from the group comprising

N,N-dimethylaminopropylamine
N,N-diethylaminopropylamine
N,N-dimethylaminoethanolamine
N,N-diethylaminoethanolamine
N,N-dimethylaminoisopropanolamine
N,N-diethylaminoisopropanolamine

**13.** Process according to claim 13, characterized in that at least one polyfunctional tertiary amine, that is to say containing a number of primary or secondary hydroxyl or amine functional groups, is combined with the monofunctional tertiary amine in a polyfunctional tertiary amine/monofunctional tertiary amine molar ratio between 2 and 200.

**14.** Process according to claim 13, characterized in that the polyfunctional tertiary amine is chosen from N,N-diethylamino-2,3-propanediol, N,N-dimethylamino-2,3-propanediol, methyldiethanolamine, ethyldiethanolamine and tri-ethanolamine.